# EUROPEAN PATENT SPECIFICATION

(11) **EP 0 592 397 B1**
(45) Date of publication and mention of the grant of the patent: **21.05.1997**
(21) Application number: 91902859.7
(22) Date of filing: 11.01.1991
(51) Int. Cl.: A61M 16/04

(54) **A RESPIRATION CATHETER**
RESPIRATIONSKATHETER
CATHETER RESPIRATOIRE

(30) Priority: 12.01.1990 DK 89/90
(43) Date of publication of application: 20.04.1994
(73) Proprietor: LOMHOLT, Vagn Niels Finsen, DK-3450 Allerod (DK)
(72) Inventor: LOMHOLT, Vagn Niels Finsen, DK-3450 Allerod (DK)
(74) Representative: Wittrup, Flemming
(86) International application number: DK9100010
(87) International publication number: WO9110464

(56) References cited:
- DK-B- 111 149
- US-A- 3 794 036

## Description

The invention concerns a respiration catheter of the type stated in the introductory portion of claim 1. Such a respiration catheter is known from DK-C-111149.

Endotracheal tubes intended for insertion through the mouth, nose or implanted in the neck (oro-naso and tracheostomy tubes) are usually provided with an inflatable cuff for sealing against the tracheal wall. The efficiency of the sealing is determind by the magnitude of the cuff pressure against the tracheal wall since the inflated cuff does not seal off pressures exceeding the pressure of the cuff against the wall. The air pressure in the cuff determines the pressure against the tracheal wall. The pressure of the cuff against the tracheal wall can be controlled and regulated only if the cuff has a sufficiently large diameter to make contact with the tracheal wall without any stretching of the sheet material of the cuff, i.e. the cuff must be lying folded on the tracheal wall. If this demand is met, the pressure in the cuff is identical with its pressure on the wall.

If the pressure of the cuff against the tracheal wall is considerably higher than 30 cm H₂O, the blood supply to the mucosa is occluded, and this causes damage in the form of superficial or deeper ulcerations after some time. This damage is prevented in that the sealing cuff, lying folded on the wall, is kept inflated from an outer pressure source with a constant, regulated pressure of 20 to 30 cm H₂O.

The sealing cuff has the additional function of preventing liquid (blood, saliva, vomit) from flowing past the cuff down into the lungs. It has been found that this function is accomplished when the pressure of the sealing cuff against the tracheal wall is at least 20 to 30 cm H₂O.

Spontaneous changes in the diameter of the trachea, changes in the catheter position and the diffusion of certain anaesthetic gases through the wall of the sealing cuff may cause considerable changes in the pressure in the sealing cuff if the inflation pressure is not controlled and regulated.

During artificial respiration the necessary pressure of the respiration air may often exceed 20 to 30 cm H₂O, and the pressure in the sealing cuff is then too low to seal off the pressure of the respiration air.

In order for the sealing cuff to be able to seal off high inflation pressures during artificial respiration, the respiration catheter described in DK-C-111149 is equipped with a two-way cut-off valve mounted on the air supply tube for the sealing cuff. In this embodiment closing and opening of the valve are controlled by the pressure changes in the external opening of the catheter tube which are produced during artifical respiration. When the pressure in the catheter tube during inflation exceeds the constant, regulated pressure of 20 to 30 cm H₂O, the cut-off valve closes so that the air in the sealing cuff cannot escape through the air supply tube, and the pressure increase in the tracheal tube is transferred to the sealing cuff, the air in the sealing cuff being compressed so that the sealing cuff can seal off the pressure prevailing in the trachea. When, during expiration, the pressure in the catheter tube decreases to 20 to 30 cm H₂O, the cut-off valve opens again, providing free connection between the source of compressed air and the sealing cuff, which then remains inflated by the constant, regulated pressure of 20 to 30 cm H₂O.

The respiration catheter according to DK-C-111149 which also forms the preamble of Claim 1 has the closing valve arranged in a respiration air supply tube at the external opening of the catheter. Due to the flow resistance between the two ends of the catheter and the consequent difference in pressure, the valve closes prematurely during inflation and opens prematurely during expiration. This entails that part of the air in the sealing cuff escapes during the expiration phase so that, for a short moment, the cuff cannot provide an efficient sealing of the trachea.

The object of the invention is to provide an embodiment of a respiration catheter of the present type which prevents the said malfunction involving the risk of liquid flowing past the sealing cuff down into the lungs.

This object is obtained in that the respiration catheter is constructed as stated in the characterizing portion of claim 1 since, in this structure, a pressure drop in the catheter tube cannot at any time cause premature opening of the closing valve and, consequently, neither undesirable escape of air from and thereby defective sealing capacity of the cuff.

An appropriate position of the cut-off valve is stated in claim 2, and claim 3 defines constructive means for establishing the desired cooperation between air supply tube, valve and sealing cuff.

When the respiration cathether is constructed as stated in claim 4, one of the two supply tube parts of this structure may be used for sucking liquid which accumulates above the sealing cuff.

Claim 5 defines a simple and inexpensive embodiment of the cut-off valve. When such a valve is arranged on the outer side of the catheter tube, movement of this tube into contact with the tracheal wall may cause unintended closing of the valve. This drawback may be avoided by constructing the valve as stated in claim 6, so that the air may follow the long way round in case the short path between the two openings is obstructed.

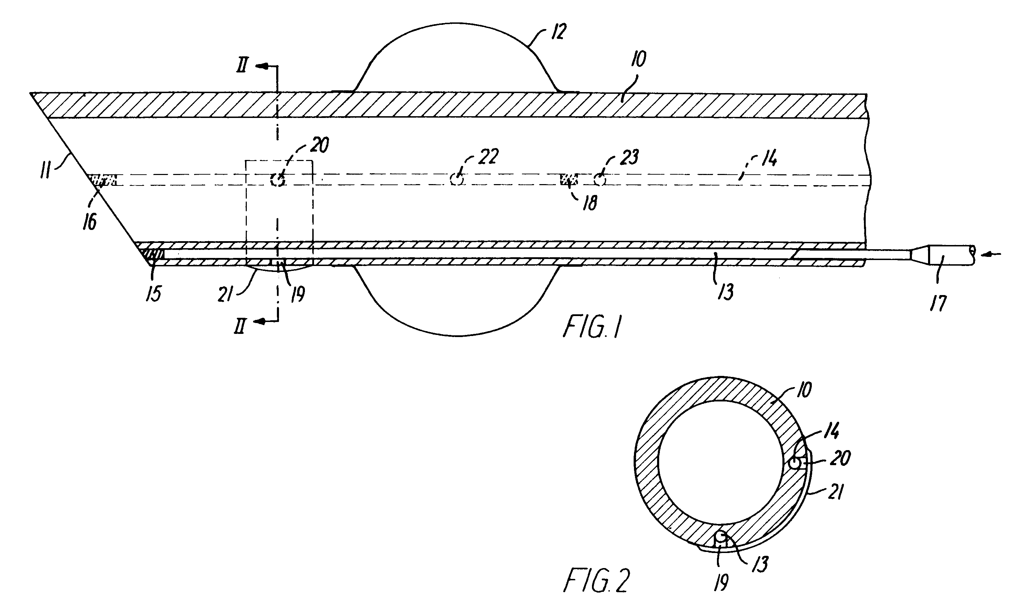

An embodiment of the respiration catheter of the invention is shown schematically in the drawing partly in longitudinal section, fig. 1, partly in cross section, fig. 2, along the line II-II in fig. 1, and will be described more fully below with reference to the drawing.

In the drawing, 10 is the end of a catheter tube intended for introduction into a patient's trachea. Close to the obliquely cut end of the tube 11 is placed a sealing cuff 12, which is made of a thin, preferably inelastic sheet material and encircles the tube. Compressed air can be supplied to the cuff through a tubing system consisting of two channels 13 and 14, which are provided in the wall of the catheter tube 10, and which are both closed by a plug 15 and 16, respectively, at the end of the tube 10. One channel 13 can be connected through a tube 17 to a source of compressed air (not shown) which delivers air with a substantially constant pressure, which may e.g. be of the order of 20 to 30 cm H₂O. Between the end 11 of the catheter tube 10 and the adjoining end of the cuff 12, each of the channels 13 and 14 are provided with a hole 19 and 20, respectively, opening to the outside of the catheter tube, and these holes are covered by a rectangular piece 21 of sheet, which is welded along the edges to the catheter tube 10 and thus forms a valve which, controlled by the pressure conditions, can establish connection between the two holes 19 and 20 and interrupt this connection. The channel 14 communicates with the interior of the cuff 12 through another hole 22 positioned between the hole 20 and a plug 18. This channel additionally has a third hole 23 which is positioned immediately beyond the end of the cuff 12 facing away from tube end 11 and such that the plug 18 will be situated between the holes 22 and 23. This hole 23 opens to the outer side of the catheter tube 10.

When the shown catheter is introduced into a patient's trachea, air is conducted from the source of compressed air through the channel 13, the valve 19, 20, 21, and the channel 14 and hole 22 to the cuff 12, which is thereby inflated and establishes sealing engagement with the wall of the trachea. The pressure applied for this purpose is so low that it cannot damage the mucuous membrane of the trachea. When, during blowing of respiration air, the pressure at the end 11 of the catheter tube 10 exceeds the pressure from the source of compressed air, the sheet 21 closes the holes 19 and 20 so that the air in the cuff cannot escape. The respiration pressure now acts on the cuff side facing the lungs and increases the engagement pressure of the cuff. This increased engagement pressure, which may be damaging if applied for a prolonged period, is applied only during the short periods when the respiration pressure exceeds the pressure from the source of compressed air.

Accordingly, the respiration catheter of the invention ensures effective sealing against the tracheal wall under all circumstances, also in case of lungs of low compliance requiring a high respiration pressure, and also ensures full perfusion of the tracheal wall since the engagement pressure is below the value which may cause damage, except for short moments.

The part of this channel positioned between the cuff 12 and the free end (not shown) of the channel 14 may be used for sucking liquid from the space between the catheter tube and the patient's trachea by connecting the free end with a suitable suction means (not shown).

The invention is not restricted to the special embodiment shown and described above. Instead of the rectangular sheet 21, a sheet ring may optionally be used, surrounding the catheter tube and welded to the tube along the edges. This establishes two paths between the holes 19 and 20, and if the short path is blocked, e.g. in that the catheter tube engages the tracheal wall, air may pass the long way round the circumference of the catheter tube.

Another possibility is to arrange the holes 19 and 20 in the inwardly facing side of the channels 13 and 14 so that they open to the interior of the catheter tube, and consequently place the sheet 21 on the inner side of the catheter tube.

The cut-off valve may also be constructed differently from what is described above and may e.g. be formed by a sleeve or bladder of thin sheet material which connects the two ends of the channels 13 and 14 with each other. Also the shape and position of the channels may differ from what is shown and described.

## Claims

1. A respiration catheter having a tube (10) intended for introduction into a patient's trachea, encircled by a sealing cuff (12) which can be connected with a source of compressed air through a separate tube (13, 14) accommodating a cut-off valve (19, 20, 21) which is controlled by the pressure of the respiration air, **characterized** in that the cut-off valve (19, 20, 21) is positioned in the vicinity of the catheter tube end (11) facing the patient's lungs.

2. A respiration catheter according to claim 1, **characterized** in that the cut-off valve (19, 20, 21) is positioned on the sealing cuff side facing the lungs.

3. A respiration catheter according to claim 1 or 2, **characterized** in that said separate tube is divided into two parts (13, 14), one (13) connected with the source of compressed air, the other (14) communicating with the sealing cuff (12), and that the two parts are interconnected through the cut-off valve (19, 20, 21).

4. A respiration catheter according to claim 3, **characterized** in that the tube part (14) connecting with the sealing cuff (12) has an extension towards the air supply end of the catheter tube (10), and that said tube part, outside and in the vicinity of the sealing cuff end facing the same way, has an opening (23) to the outer side of the catheter tube and is closed between this opening and the connection with the sealing cuff preferably by a plug (18) positioned in the tube part.

5. A respiration catheter according to claim 3 or 4, **characterized** in that the cut-off valve is formed by a piece of sheet (21) which is so placed across the openings (19, 20) in the two tube parts (13, 14) that, in unactivated state, it allows connection between these two parts and interrupts this connection in activated state.

6. A respiration catheter according to claim 5, **characterized** in that the sheet (21) is ring-shaped and surrounds the catheter tube (10).

7. A respiration catheter according to any of claims 1-5, **characterized** in that the cut-off valve (19, 20, 21) is positioned in the catheter tube (10).

## Patentansprüche

1. Respirationskatheter mit einem Tubus (10), der zum Einführen in die Trachea eines Patienten vorgesehen ist und von einer Dichtungsmanschette (12) umfaßt ist, die mit einer Druckluftquelle über eine separate Leitung (13, 14) verbunden werden kann, die ein Absperrventil (19, 20, 21) aufweist, das von dem Druck der Respirationsluft gesteuert wird, dadurch gekennzeichnet, daß das Absperrventil (19, 20, 21) in der Nähe des Kathetertubusendes (11), das den Lungen des Patienten zugewandt ist, angeordnet ist.

2. Respirationskatheter nach Anspruch 1, dadurch gekennzeichnet, daß das Absperrventil (19, 20, 21) an der den Lungen zugewandten Seite der Dichtungsmanschette angeordnet ist.

3. Respirationskatheter nach Anspruch 1 oder 2, dadurch gekennzeichnet, daß die separate Leitung in zwei Teile (13, 14) unterteilt ist, wobei das eine Teil (13) mit der Druckluftquelle verbunden ist und das andere Teil (14) mit der Dichtungsmanschette (12) kommuniziert, und daß die beiden Teile durch das Absperrventil (19, 20, 21) miteinander verbunden sind.

4. Respirationskatheter nach Anspruch 3, dadurch gekennzeichnet, daß das mit der Dichtungsmanschette (12) verbundene Leitungsteil (14) eine Verlängerung in Richtung zum Luftversorgungsende des Kathetertubus (10) aufweist, und daß dieses Leitungsteil außerhalb und in der Nähe des in die gleiche Richtung weisenden Dichtungsmanschettenendes eine Öffnung (23) zur Außenseite des Kathetertubus aufweist und zwischen dieser Öffnung und der Verbindung mit der Dichtungsmanschette vorzugsweise durch einen Stopfen (18), der in dem Leitungsteil angeordnet ist, verschlossen ist.

5. Respirationskatheter nach Anspruch 3 oder 4, dadurch gekennzeichnet, daß das Absperrventil von einem Stück einer Folie (21) gebildet ist, die derart auf die Öffnungen (19, 20) in den beiden Leitungsteilen (13, 14) gelegt ist, daß sie in nichtaktiviertem Zustand eine Verbindung zwischen diesen zwei Teilen gestattet und in aktiviertem Zustand diese Verbindung unterbricht.

6. Respirationskatheter nach Anspruch 5, dadurch gekennzeichnet, daß die Folie (21) ringförmig ist und den Kathetertubus (10) umgibt.

7. Respirationskatheter nach einem der Ansprüche 1 bis 5, dadurch gekennzeichnet, daß das Absperrventil (19, 20, 21) in dem Kathetertubus (10) angeordnet ist.

## Revendications

1. Cathéter respiratoire possédant un tube (10) prévu pour être introduit dans la trachée d'un patient, entouré d'un manchon d'étanchéité (12) qui peut être raccordé à une source d'air comprimé par l'intermédiaire d'un tube distinct (13, 14) logeant une vanne d'arrêt (19, 20, 21) qui est commandée par la pression de l'air respiré, caractérisé en ce que la vanne d'arrêt (19, 20, 21) est placée à proximité de l'extrémité du tube de cathéter (11) dirigée vers les poumons du patient.

2. Cathéter respiratoire selon la revendication 1, caractérisé en ce que la vanne d'arrêt (19, 20, 21) est placée du côté du manchon d'étanchéité dirigé vers les poumons.

3. Cathéter respiratoire selon la revendication 1 ou 2, caractérisé en ce que ledit tube distinct est divisé en deux parties (13, 14), l'une (13) étant raccordée à la source d'air comprimé, l'autre (14) communiquant avec le manchon d'étanchéité (12), et en ce que les deux parties sont raccordées l'une à l'autre par la vanne d'arrêt (19, 20, 21).

4. Cathéter respiratoire selon la revendication 3, caractérisé en ce que la partie de tube (14) se raccordant au manchon d'étanchéité (12) possède une extension dirigée vers l'extrémité d'alimentation en air du tube de cathéter (10), et en ce que ladite partie de tube, à l'extérieur et à proximité de l'extrémité de manchon d'étanchéité dirigée de la même manière, possède une ouverture (23) donnant sur le côté extérieur du tube de cathéter, et est fermée entre cette ouverture et le raccordement au manchon d'étanchéité, de préférence par un bouchon (18) placé dans la partie de tube.

5. Cathéter respiratoire selon la revendication 3 ou 4, caractérisé en ce que la vanne d'arrêt est formée par une feuille (21) qui est placée de manière à recouvrir les ouvertures (19, 20) dans les deux parties de tube (13, 14) de telle sorte que, à l'état de repos, elle permet le raccordement de ces deux parties entre elles et interdit ce raccordement à l'état actif.

6. Cathéter respiratoire selon la revendication 5, caractérisé en ce que la feuille (21) a une forme annulaire et entoure le tube de cathéter (10).

7. Cathéter respiratoire selon l'une quelconque des revendications 1 à 5, caractérisé en ce que la vanne d'arrêt (19, 20, 21) est placée dans le tube de cathéter (10).
